# DEMANDE DE BREVET EUROPEEN

(11) **EP 1 661 554 A1**
(43) Date de publication de la demande: **31.05.2006**
(21) Numéro de dépôt: 05111199.5
(22) Date de dépôt: 24.11.2005
(51) Int. Cl.: A61K 8/90, A61Q 5/06, A61Q 5/10

(54) **Composition aqueuse de coloration des fibres kératiniques comprenant un colorant et un copolymère blocs particulier**

(30) Priorité: 25.11.2004 FR 0452761
(71) Demandeur: L'OREAL, 75008 Paris (FR)
(72) Inventeur: Giroud, Franck, 92110 Clichy (FR); Panangatte, Lydia, 78300 Poissy (FR); Dubief, Claude, 78150 Le Chesnay (FR)
(74) Mandataire: Poulin, Gérard

(57) **Abrégé**

L'invention a trait à une composition de coloration des fibres kératiniques comprenant un milieu aqueux physiologiquement acceptable comprenant :
- au moins un colorant porteur d'au moins un groupe ionique, ledit colorant étant présent en une teneur allant de 0,001 à 5% en poids par rapport au poids total de la composition ;
- au moins un copolymère blocs comprenant au moins un bloc hydrosoluble non ionique et au moins un bloc ionique présentant des charges opposées à celle de la charge ionique portée par le colorant.

## Description

### DOMAINE TECHNIQUE

La présente invention concerne une composition aqueuse de coloration des fibres kératiniques, en particulier des fibres kératiniques humaines et, plus particulièrement, des cheveux, qui comprend au moins un colorant porteur d'une charge ionique et un copolymère blocs particulier.

L'invention concerne, en outre, un procédé de coloration des fibres kératiniques, en particulier des fibres kératiniques humaines et, plus particulièrement, des cheveux, qui met en oeuvre ladite composition.

### ÉTAT DE LA TECHNIQUE ANTÉRIEURE

L'industrie des matières colorantes commercialise un grand nombre de molécules qui trouvent leur utilisation dans divers domaines industriels. Parmi ces molécules, certaines porteuses de groupements anioniques comme le groupe sulfonate, carboxylate, de groupements cationiques comme les groupes ammoniums primaires, secondaires et tertiaires, sont, du fait de leur affinité avec la kératine et de leurs résultats tinctoriaux, utilisées dans la formulation de produits de coloration capillaire.

Ces molécules peuvent être introduites dans des véhicules aqueux ce qui, après application sur les cheveux, permettra leur transfert. Dans ce cas, il est souhaitable que les colorants soient et restent solubles dans les véhicules considérés, montent rapidement sur les cheveux mais de façon uniforme quelle que soit leur sensibilisation et résistent aux actions répétées des lavages et des expositions à la lumière.

Ces molécules peuvent être également introduites dans des produits de coloration d'oxydation afin d'en moduler les nuances. Dans ce cas, il est souhaitable que les colorants, en plus des propriétés mentionnées ci-dessus, présentent une résistance au milieu oxydant alcalin qui caractérise ces produits.

Il s'avère à l'heure actuelle que les colorants porteurs de groupe(s) ionique(s) ne présentent pas des propriétés totalement satisfaisantes.

Or, après de longues recherches menées sur la question, la Demanderesse a découvert que, de manière surprenante, l'association d'un copolymère blocs particulier avec un colorant porteur d'un groupe ionique permet d'augmenter la solubilité en milieu aqueux et/ou la stabilité en milieu oxydant et réducteur, et/ou la montée et/ou la chromaticité sur cheveux d'un colorant ionique. La Demanderesse a également découvert qu'une telle association facilitait l'unisson, c'est-à-dire la répartition homogène de la coloration le long d'une même fibre kératinique et entre les fibres kératiniques. Et c'est cette découverte qui est à la base de l'invention.

### EXPOSÉ DE L'INVENTION

Ainsi, l'invention a, en premier lieu, pour objet, une composition de coloration des fibres kératiniques, comprenant un milieu aqueux physiologiquement acceptable, comprenant .
- au moins un colorant porteur d'au moins un groupe ionique, ledit colorant étant présent en une teneur allant de 0,001 à 5% en poids par rapport au poids total de la composition ;
- au moins un copolymère blocs comprenant au moins un bloc non ionique hydrosoluble et au moins un bloc ionique présentant des charges opposées à celle de la charge ionique portée par le colorant.

Selon l'invention, les colorants peuvent être choisis parmi les colorants directs acides et les colorants directs basiques.

Rappelons qu'un colorant direct est une substance colorée qui ne nécessite pas l'emploi d'un agent oxydant pour révéler sa couleur. Dans la suite de cet exposé, on utilisera les termes colorants acides et colorants basiques.

On précise que par colorant acide, on entend généralement un colorant comportant au moins un groupe COOH, SO₃H, PO₃H, ou PO₄H₂, lesdits groupes pouvant exister sous forme de sels.

On précise que, par sels, on entend généralement des sels de métaux (par exemple, métaux alcalins ou alcalino-terreux), des sels d'amine organique éventuellement hydroxylée.

Ces colorants sont également connus sous l'appellation de colorants anioniques.

On précise, de plus, que par colorants basiques, on entend un colorant présentant au moins un groupe porteur d'une charge positive, tel qu'un groupe ammonium ou un atome d'azote quaternisé dans un cycle.

Peuvent également convenir dans la présente invention, à titre de colorant basique, bien que de manière non préférée, les colorants directs possédant une ou plusieurs amines éventuellement substituées, non directement liées à un noyau aromatique ou hétérocyclique.

Ces colorants sont également connus sous l'appellation de colorants cationiques.

Les colorants acides pouvant être utilisés dans le cadre de cette invention peuvent être choisis parmi les colorants nitrés acides, les colorants azoïques acides, les colorants aziniques acides, les colorants triarylméthaniques acides, les colorants quinoniques acides, les colorants indoaminiques acides les colorants naturels acides et les mélanges de ceux-ci.

A titre illustratif, on peut citer les colorants suivants :
- Sel de sodium de l'acide 2,4-dinitro-1-naphtol-7-sulfonique ;
- Acid Orange 3;
- Acid Yellow 9 / Food Yellow 2;
- Direct Red 45 / Food Red 13;
- Acid Black 52;
- Acid Yellow 36;
- Sel de sodium de l'acide 1-hydroxy-2-(2',4'-xylyl-5-sufonatoazo)-naphtalène-4-sulfonique /Food Red 1 ;
- Acid Red 14/ Food Red 3/ Mordant Blue 79;
- Acid Red 18;
- Acid Brown 4;
- Acid Orange 7 / Pigment Orange 17 / Solvent Orange 49;
- Food Yellow 3 / Pigment Yellow 104;
- Acid Red 27 / Food Red 9;
- Acid Orange 10 / Food Orange 4;
- Acid Red 44;
- Acid Red 33 / Food Red 12;
- Sel de sodium de l'acide 1-(3'-nitro-5'-sulfo-6'-oxophénylazo)-8-acétamido-naphtalène-3,6-disulfonique / Food Red 11 ;
- Sel de sodium de l'acide 1-hydroxy-2-(2'-méthyl phénylazo)-8-acétamido-naphtalene-3,6-disulfonique / Acid Red 35 ;
- Acid Violet 3;
- Acid Violet 43;
- Acid Red 35;
- Acid Violet 7;
- Acid Red 135;
- Acid Yellow 27;
- Acid Yellow 23 / Food Yellow 4;
- Acid Yellow 36;
- 4'-(sulfonato-2'',4''-diméthyl)-bis-(2,6-phénylazo)-1,3-dihydroxy benzène / Acid Orange 24
- Acid Black 1 (Sel de sodium de l'acide 1-amino-2-(4'-nitrophénylazo)-7-phénylazo-8-hydroxy-naphtalène-3,6-disulfonique) ;
- (4-((4-méthylphényl) sulfonyloxy)-phényl azo)2,2'-diméthyl-4-((2-hydroxy-5,8-disulfonato)naphtylazo)biphényle / Acid Red 111 ;
- Food Black 2 ;
- 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 ;
- Acide 4-β-hydroxyéthylamino-3-nitro benzènesulfonique ;
- Acid Blue 9 ;
- (5',6' ou 7')-sulfonato-6'-méthylquinoléine-2,2',Δ-1,3-indanedione / Acid Yellow 3 ;
- Sel de sodium de l'acide 4-hydroxy-3((2-méthoxy phényl)-azo)-1-naphtalène sulfonique / Acid Red 4 ;
- Acide 2-pipéridino 5-nitro benzène sulfonique ;
- Acide 2(4'-N,N(2"-hydroxyéthyl)amino-2'-nitro)aniline éthane sulfonique ;
- Acide 4-β-hydroxyéthylamino-3-nitrobenzène sulfonique ;
- Acid Violet 49 ;
- Acid Blue 7;
- Acid Blue 156;
- Acid Blue 317;
- Acid Blue 62.

La plupart de ces colorants sont decrits, en particulier, dans le Color Index publié par The Society of Dyers and Colorists, P.O.Box 244, Perkin House, 82 Grattant Road, Bradford, Yorkshire, BD1 2JBN England.

Les colorants basiques pouvant être utilisés dans le cadre de cette invention peuvent être choisis parmi les colorants benzéniques nitrés, les colorants azoïques, les colorants azométhiniques, les colorants méthiniques, les colorants tétraazapentaméthiniques, les colorants anthraquinoniques, les colorants naphtoquinoniques, les colorants benzoquinoniques, les colorants phénotiaziniques, les colorants indigoïdes, les colorants xanthéniques, les colorants phénanthridiniques, les colorants phtalocyanines, les colorants dérivés du triarylaméthane, les colorants naturels basiques et les mélanges de ceux-ci.

Parmi les colorants directs utilisables selon l'invention on peut citer les colorants cationiques décrits dans les demandes de brevets WO 95/15144, WO 95/01772, EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants .
- chlorure de 1,3-diméthyl-2-[[4-(diméthyl amino)phényl] azo]-1H-Imidazolium ;
- chlorure de 1,3-diméthyl-2-[(4-aminophényl)azo]-1H-Imidazolium ;
- méthylsulfate de 1-méthyl-4-[(méthylphényl hydrazono) méthyl]-pyridinium ;
- Basic Red 22;
- Basic Red 76;
- Basic Yellow 57;
- Basic Brown 16;
- Basic Blue 41;
- Basic Blue 67;
- Basic Brown 1;
- Basic Brown 4;
- Basic Red 18;
- Basic Red 46;
- Basic Red 104;
- Basic Red 118;
- Basic Violet 35;
- Basic Yellow 45;
- Basic Yellow 67;
- Basic Red 14;
- Basic Yellow 13;
- Basic Yellow 29;
- Basic Brown 17;
- Basic Blue 22;
- Basic Blue 99 ;
- 1-N-méthylmorpholiniumpropylamino-4-hydroxyanthraquinone ;
- Basic Blue 17 ;
- Basic Red 2 ;
- Basic Green 1;
- Basic Violet 3;
- Basic Violet 14;
- Basic Blue 7;
- Basic Blue 26.

Il est entendu que le bloc ionique du copolymère blocs sera un bloc anionique, dans le cas où le colorant sera un colorant cationique (ou colorant direct basique), et que le bloc ionique du copolymère blocs sera un bloc cationique, dans le cas où le colorant sera un colorant anionique (ou colorant direct acide).

Selon l'invention, on précise que par bloc non ionique hydrosoluble, on entend généralement un bloc polymère comprenant au moins 80% en moles d'au moins un monomère hydrosoluble et éventuellement jusqu'à 20% en moles d'un ou plusieurs monomères insolubles dans l'eau, ce ou ces monomères insolubles dans l'eau étant répartis de façon aléatoire au sein du bloc hydrosoluble. Cette proportion de monomères insolubles dans l'eau est de préférence au plus égale à 10% en moles et, mieux encore, inférieure ou égale à 5% en moles.

On précise que, par composé hydrosoluble (polymère ou monomère), on entend généralement un composé qui, introduit dans l'eau à 25°C, et si besoin neutralisé, à une concentration en poids égale à 0,1%, permet l'obtention d'une solution ou d'une suspension macroscopiquement homogène et transparente, c'est-à-dire ayant une valeur de transmittance de la lumière, à une longueur d'onde égale à 500 nm, à travers un échantillon de 1 cm d'épaisseur, d'au moins 70%, de préférence d'au moins 80%.

De préférence, les copolymères blocs utilisés dans les compositions de l'invention sont dénués d'unités saccharidiques.

Les blocs anioniques peuvent résulter avantageusement de la polymérisation d'un ou plusieurs monomères choisis parmi les monomères porteurs d'au moins une fonction acide carboxylique, les monomères porteurs d'au moins une fonction acide sulfonique, les monomères porteurs d'au moins une fonction acide phosphonique et les mélanges de ceux-ci.

Des monomères porteurs de fonction(s) carboxylique(s) appropriés peuvent être choisis parmi l'acide (méth)acrylique, l'acide aspartique, l'acide 1,4-phénylènediacrylique, l'acide citraconique, l'acide trans-cinammique, l'acide 4-hydroxy-3-méthoxycinnamique, l'acide p-hydroxycinnamique, l'acide trans-glutaconique, l'acide glutamique, l'acide itaconique, l'acide linoléique, l'acide linolénique, l'acide maléique, l'acide méthylfumarique, l'acide trans-hex-3-ène dioïque, l'acide trans-trans muconique, l'acide oléique, l'acide ricinoléique, l'acide trans-traumatique, l'acide vinylbenzoïque, l'acide vinylglycolique.

Des monomères porteurs de fonction(s) sulfonique(s) appropriés peuvent être choisis parmi l'acide 2-propène-1-sulfonique, l'acide acrylamidopropane sulfonique, l'acide 4-styrènesulfonique et l'acide vinylsulfonique.

Des monomères porteurs de fonction(s) phosphonique(s) appropriés peuvent être l'acide vinylphosphonique.

Il est entendu que les monomères listés ci-dessus peuvent exister sous forme de sels, tels que des sels de sodium, de potassium ou d'ammonium.

Selon l'invention, le bloc anionique de l'invention est avantageusement un bloc résultant de la polymérisation de l'acide méthacrylique éventuellement sous forme de sels et encore plus préférentiellement de l'acide acrylique éventuellement sous forme de sels.

Les blocs cationiques peuvent être des blocs de polyaminoacides(tels que la polylysine), des blocs d'esters alkanolamines de l'acide polyméthacrylique (tels que le poly(diméthylammonioéthylméthacrylate), des blocs de polyamines (telles que la polyspermine, le polyéthylèneimine) ou des blocs de polyvinylpyridine.

Les blocs non ioniques hydrosolubles comme mentionnés ci-dessus comprennent une large proportion de monomères hydrosolubles non-ioniques et éventuellement des monomères hydrophobes non-ioniques en faibles proportions.

Les monomères hydrosolubles non-ioniques peuvent être choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, le méthacrylate d'hydroxyéthyle et le méthacrylate d'hydroxypropyle.

Les monomères non-ioniques insolubles dans l'eau peuvent être choisis parmi les monomères vinylaromatiques alkylés ou non, tels que le styrène et les styrènes alkylés comme le 4-butylstyrène, l'a-méthylstyrène et le vinyltoluène ; les diènes tels que le butadiène et le 1,3-hexadiène, et les diènes alkylés tels que l'isoprène et le diméthylbutadiène ; le chloroprène ; les acrylates d' alkyle en C₁₋₁₀, d'aryle en C₆₋₁₀ ou d'aralkyle en C₆₋₁₀, comme par exemple les (méth)acrylates de méthyle, d'éthyle, de n-butyle, de 2-éthylhexyle, de tert-butyle, d'isobornyle, de phényle ou de benzyle ; l'acétate de vinyle ; les vinyléthers de formule CH₂=CH-CH₂-O-R dans laquelle R représente un groupe alkyle en C₁₋₆ ; l'acrylonitrile ; le chlorure de vinyle ; le chlorure de vinylidène ; la caprolactone ; l'éthylène ; le propylène ; les monomères vinyliques fluorés ou à chaîne perfluorée, tels que les acrylates et méthacrylates de fluoroalkyle ou les α-fluoroacrylates d'alkyle.

De préférence, les monomères insolubles dans l'eau sont choisis parmi les composés vinyliques aromatiques tels que le styrène, le 4-butylstyrène, l'α-méthylstyrène et le vinyltoluène, le styrène étant particulièrement préféré.

La masse moléculaire moyenne en nombre de chaque bloc du copolymère blocs va de préférence de 500 à 10⁶, de préférence de 800 à 500 000.

Des copolymères particuliers susceptibles d'être utilisés dans le cadre de l'invention sont le poly(méthylsulfate d'acryloxyéthyl-triméthylammonium-b-acrylamide), qui sera utilisé avec un colorant anionique, et le poly(acrylate de sodium-b-acrylamide), qui sera utilisé avec un colorant cationique.

Les copolymères blocs de l'invention peuvent être préparés par les procédés de synthèse classiquement utilisés pour obtenir des polymères blocs. On peut citer par exemple la polymérisation anionique et la polymérisation radicalaire contrôlée (voir « New Method of Polymer Synthesis », Blackie Academic & Professional, Londres, 1995, volume 2, page 1, ou Trends Polym. Sci.4, page 183 (1996) de C.J Hawker) qui peut être mise en oeuvre suivant différents procédés comme, par exemple, la polymérisation radicalaire par transfert d'atomes (Atom Transfert Radical Polymerisation ou ATRP) (voir JACS, 117, page 5614 (1995), de Matyjasezwski et al.), la méthode des radicaux tels que les nitroxides (Georges et al., Macromolecules, 1993, 26, 2987).

On peut aussi utiliser ces procédés pour obtenir un seul des deux types de blocs du copolymère de l'invention, l'autre bloc étant introduit dans le polymère final par l'intermédiaire de l'amorceur utilisé ou bien par réaction de couplage entre les deux blocs.

Les copolymères blocs de l'invention sont généralement présents en une quantité allant de 0,001 à 20% en poids par rapport au poids total de la composition, préférentiellement de 0,01 à 10% en poids.

Outre la présence d'un colorant et d'un polymère blocs tel que défini ci-dessus, les compositions de l'invention comprennent également un milieu aqueux cosmétiquement acceptable, c'est-à-dire un milieu compatible avec les fibres kératiniques, telles que les cheveux.

La composition peut comprendre également au moins une base d'oxydation. Cette base d'oxydation est avantageusement choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

La composition peut de même contenir un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.

Généralement, la teneur en base d'oxydation, en coupleur, lorsque ces composés sont présents, est comprise entre 0,001 et 10 % en poids du poids total de la composition tinctoriale, et plus préférentiellement de 0,005 à 6 % en poids.

D'une manière générale, les sels d'addition avec un acide utilisables sont notamment choisis parmi les chlorhydrates, les bromhydrates, les sulfates, les citrates, les succinates, les tartrates, les lactates, les tosylates, les benzènesulfonates, les phosphates et les acétates.

Le milieu aqueux cosmétiquement acceptable est généralement constitué d'eau ou d'un mélange d'eau et d'au moins un solvant organique choisi parmi les alcools inférieurs en C₁-C₄, tels que l'éthanol, l'isopropanol, le tertio-butanol ou le n-butanol ; les polyols tels que glycérol, le propylèneglycol et les polyéthylèneglycols.

Le pH des compositions selon l'invention est généralement compris entre 4 et 11.

Le pH peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 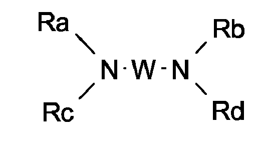 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition selon l'invention peut comprendre également au moins un agent oxydant. La présence d'un tel agent peut être notamment utile pour obtenir différentes nuances de couleur à partir d'un colorant donné.

Les agents oxydants utilisables sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition selon l'invention peut comprendre en outre des additifs choisis parmi les tensioactifs anioniques, non-ioniques, amphotères, les polymères filmogènes anioniques, non ioniques différents des polymères blocs décrits ci-dessus, les polymères conditionneurs cationiques ou amphotères, les silicones linéaires, ramifiées ou cycliques, volatiles ou non, organomodifiées ou non, les épaississants polymères associatifs ou non, les épaississants non polymères, les agents nacrants, les opacifiants, les colorants ou les pigments, les parfums, les huiles minérales, végétales et/ou synthétiques, les cires, les vitamines, les filtres UV, les agents anti-radicalaires, les antipelliculaires, les conservateurs, les agents de stabilisation de pH, les solvants et leurs mélanges.

L'homme du métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20% en poids par rapport au poids total de la composition.

Ces compositions peuvent constituer des compositions de coloration directe, c'est-à-dire ne nécessitant pas de révélation par des agents révélateurs tel que agents oxydants.

Ainsi, un autre objet de la présente invention est un procédé de coloration directe des fibres kératiniques comprenant l'application d'au moins une composition de teinture telle que définie ci-dessus sur des fibres sèches ou humides puis l'observation d'un temps de pause suivi éventuellement du rinçage des fibres.

Le temps de pause est généralement compris entre quelques secondes et 30 minutes, de préférence entre 3 et 15 minutes.

La température à laquelle la composition est laissée agir, est, en général, comprise entre 15 et 220°C, plus particulièrement entre 15 et 80°C, de préférence entre 15 et 40°C.

Les compositions de l'invention peuvent être également des compositions à mélanger avec des compositions de coloration d'oxydation afin d'en moduler les propriétés tinctoriales ou faire partie intégrante d'une composition de coloration d'oxydation.

Les compositions selon l'invention, même exemptes de colorant d'oxydation (base d'oxydation/coupleur) peuvent aussi être mises en oeuvre en présence d'un agent oxydant.

Dans ce cas, on applique sur les fibres la composition selon l'invention, en présence d'un agent oxydant qui peut soit être mélangé à la composition selon l'invention (pour obtenir une composition prête à l'emploi), soit appliqué directement sur la fibre kératinique.

De manière classique, l'agent oxydant est choisi parmi les composés décrits auparavant. La teneur en agent oxydant est en général comprise entre 1 et 40 % en poids, par rapport au poids de la composition prête à l'emploi, de préférence entre 1 et 20 % en poids.

Généralement, la composition comprenant l'agent oxydant utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

Le pH de la composition peut être réglé au moyen d'un agent alcalinisant ou acidifiant, notamment choisis parmi ceux mentionnés auparavant, dans le cadre de la description selon l'invention.

La composition en présence de l'agent oxydant est donc appliquée sur des fibres sèches ou humides, pendant un temps de pause suffisant pour obtenir la coloration recherchée.

Le temps de pause et la température à laquelle la composition est laissée agir sont du même ordre que ceux du procédé mis en oeuvre en l'absence d'un agent oxydant.

A l'issue du temps de pause, la composition est éliminée par un rinçage à l'eau, suivi éventuellement d'un lavage avec un shampooing et d'un rinçage, puis éventuellement d'un séchage.

Enfin, l'invention a trait à l'utilisation d'un copolymère blocs tel que défini ci-dessus pour augmenter la solubilité en milieu aqueux de colorants tels que définis ci-dessus, la stabilité en milieu oxydant et réducteur et pour améliorer l'unisson sur les fibres kératiniques.

L'invention va maintenant être décrite en référence aux exemples qui suivent donnés à titre illustratif et non limitatif.

### EXPOSÉ DÉTAILLÉ DE MODES DE RÉALISATION PARTICULIERS

### Exemple 1

Dans cet exemple, on a procédé à la coloration de mèches de cheveux blancs permanentés à l'aide du colorant (Acid Red 18) utilisé à la concentration de 0,6 % dans l'eau à pH=9 en présence ou non de 1 % en matière active d'un copolymère diblocs poly(méthylsulfate d'acryloxyéthyl-triméthylammonium-b-acrylamide) dont chaque bloc a pour masse moléculaire respectivement 11 000 et 30 000 (dénommé P1 dans le tableau ci-dessous).

Le tableau ci-dessous illustre parfaitement la contribution du polymère diblocs défini ci-dessus en milieu basique dans l'amélioration de la montée du colorant acide.

Les mèches sont lues au moyen d'un colorimètre Minolta CM3600d dans le système C.I.E. L*a*b* (composantes spéculaires inclues, illuminant D65, angle 10°).

Plus la valeur de L* est faible, plus la couleur est intense.

| Traitement | L* |
|---|---|
| Cheveux blancs permanentés non colorés | 60,5 |
| Acid Red 18 à 0,6 %, pH=9 | 60,1 |
| P1 + Acid Red 18, pH=9 | 51,8 |

On constate donc que la mèche colorée en mettant en oeuvre la composition selon l'invention est significativement plus colorée que la mèche témoin et la mèche ne comprenant que le colorant acide.

De plus, la chromaticité de la mèche obtenue en mettant en oeuvre la composition selon l'invention est significativement plus importante que celle obtenue en mettant en oeuvre le colorant acide seul.

### Exemple 2.

Dans cet exemple, il a été procédé à des essais destinés à mettre en évidence l'effet protecteur des polymères blocs conformes à l'invention vis-à-vis de colorants instable en milieu oxydant alcalin.
Le colorant testé est le Basic Red 76.

Lors d'un premier essai, on a ajouté dans un tube à essai une solution ammoniacale à 0,04% en poids de colorant à pH= 9 et on a laissé reposer la solution pendant 1 heure.

Lors d'un second essai, on a ajouté dans un tube à essai une solution ammoniacale à 0,04 % en poids de colorant à pH=9 en présence d'un copolymère diblocs poly(acrylate de sodium-b-acrylamide) dont chaque bloc a pour masse moléculaire moyenne en nombre respectivement de 5000 et 30 000 et en présence d'eau oxygénée à 3% et on laisse reposer pendant 1 heure.

Pour le premier essai, on constate une dégradation du colorant au bout d'une heure, se traduisant par une perte visible de coloration de la solution.

Pour le second essai, la coloration de la solution reste stable même après 1 heure de repos, ce qui atteste de la stabilité du colorant en milieu basique oxydant lors qu'il est mis en présence d'un polymère blocs conforme à l'invention.

### EXEMPLE 3.

Dans cet exemple, il a été procédé à des essais destinés à mettre en évidence l'augmentation de la solubilité de colorants difficilement en milieu aqueux grâce à la présence de polymères blocs conformes à l'invention.

Le colorant testé est l'Astrazon Orange R (ou Basic Orange 22).

Lors d'un premier essai, on ajoute dans un tube à essai une solution aqueuse comprenant 0,04% de colorant. Cette solution présente un aspect trouble, attestant ainsi de la mauvaise solubilité de ce colorant en milieu aqueux.

Lors d'un deuxième essai, on ajoute dans un tube à essai une solution comprenant 0,04% de colorant puis 0,05% en poids d'un copolymère diblocs poly(acrylate de sodium-b-acrylamide) (copolymère de l'exemple 2). La solution résultante est une solution limpide. Ceci atteste ainsi de la capacité des polymères blocs conformes à l'invention d'augmenter la solubilité de colorants difficilement solubles en milieu aqueux.

## Revendications

1. Composition de coloration des fibres kératiniques, comprenant un milieu aqueux physiologiquement acceptable, comprenant .
- au moins un colorant porteur d'au moins un groupe ionique, ledit colorant étant présent en une teneur allant de 0,001 à 5% en poids par rapport au poids total de la composition ;
- au moins un copolymère blocs comprenant au moins un bloc hydrosoluble non ionique et au moins un bloc ionique présentant des charges opposées à celle de la charge ionique portée par le colorant.

2. Composition de coloration selon la revendication 1, dans laquelle le colorant est choisi parmi les colorants directs acides, lorsque le bloc ionique est un bloc cationique.

3. Composition de coloration selon la revendication 2, dans laquelle les colorants directs acides comportent au moins un groupe COOH, SO₃H, PO₃H, ou PO₄H₂, lesdits groupes pouvant exister sous forme de sels.

4. Composition de coloration selon la revendication 2 ou 3, dans laquelle les colorants directs acides sont choisis parmi les colorants nitrés acides, les colorants azoïques acides, les colorants aziniques acides, les colorants triarylméthaniques acides, les colorants quinoniques acides, les colorants indoaminiques acides, les colorants naturels acides et les mélanges de ceux-ci.

5. Composition de coloration selon la revendication 1, dans laquelle le colorant est choisi parmi les colorants directs basiques, lorsque le bloc ionique est un bloc anionique.

6. Composition de coloration selon la revendication 5, dans laquelle les colorants directs basiques sont choisis parmi les colorants benzéniques nitrés, les colorants azoïques, les colorants azométhiniques, les colorants méthiniques, les colorants tétraazapentaméthiniques, les colorants anthraquinoniques, les colorants naphtoquinoniques, les colorants benzoquinoniques, les colorants phénotiaziniques, les colorants indigoïdes, les colorants xanthéniques, les colorants phénanthridiniques, les colorants phtalocyanines, les colorants dérivés du triarylaméthane, les colorants naturels basiques et les mélanges de ceux-ci.

7. Composition de coloration selon l'une quelconque des revendications précédentes, dans laquelle le bloc hydrosoluble non-ionique comprend au moins 80% en moles d'au moins un monomère hydrosoluble non-ionique.

8. Composition de coloration selon l'une quelconque des revendications précédentes, dans laquelle le bloc hydrosoluble non-ionique comprend jusqu'à 20% en moles d'un ou plusieurs monomères insolubles dans l'eau, ce ou ces monomères insolubles dans l'eau étant répartis de façon aléatoire au sein du bloc hydrosoluble non-ionique.

9. Composition de coloration selon la revendication 7, dans laquelle le monomère hydrosoluble non-ionique est choisi dans le groupe constitué par l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, le méthacrylate d'hydroxyéthyle et le méthacrylate d'hydroxypropyle.

10. Composition de coloration selon la revendication 8, dans laquelle le monomère insoluble dans l'eau est choisi dans le groupe constitué par les monomères vinylaromatiques alkylés ou non, les diènes alkylés ou non, le chloroprène, les acrylates d'alkyle en C₁₋₁₀, d'aryle en C₆₋₁₀ ou d'aralkyle en C₆₋₁₀, l'acétate de vinyle, les vinyléthers de formule CH₂=CH-CH₂-O-R dans laquelle R représente un groupe alkyle en C₁₋₆ ; l'acrylonitrile ; le chlorure de vinyle ; le chlorure de vinylidène ; la caprolactone ; l'éthylène ; le propylène ; les monomères vinyliques fluorés ou à chaîne perfluorée.

11. Composition de coloration selon la revendication 5, dans laquelle le bloc anionique résulte de la polymérisation d'un ou plusieurs monomères choisis parmi les monomères porteurs d'au moins une fonction acide carboxylique, les monomères porteurs d'au moins une fonction acide sulfonique, les monomères porteurs d'au moins une fonction acide phosphonique et les mélanges de ceux-ci.

12. Composition de coloration selon la revendication 11, dans lequel les monomères porteurs d'au moins une fonction acide carboxylique sont choisis dans le groupe constitué par l'acide (méth)acrylique, l'acide aspartique, l'acide 1,4-phénylènediacrylique, l'acide citraconique, l'acide trans-cinammique, l'acide 4-hydroxy-3-méthoxy-cinnamique, l'acide p-hydroxycinnamique, l'acide trans-glutaconique, l'acide glutamique, l'acide itaconique, l'acide linoléique, l'acide linolénique, l'acide maléique, l'acide méthylfumarique, l'acide trans-hex-3-ène dioïque, l'acide trans-trans muconique, l'acide oléique, l'acide ricinoléique, l'acide trans-traumatique, l'acide vinylbenzoïque, l'acide vinylglycolique.

13. Composition de coloration selon la revendication 11, dans lequel les monomères porteurs d'au moins une fonction acide sulfonique sont choisis dans le groupe constitué par l'acide 2-propène-1-sulfonique, l'acide acrylamidopropane sulfonique, l'acide 4-styrènesulfonique, l'acide vinylsulfonique.

14. Composition de coloration selon la revendication 2, dans laquelle le bloc cationique est choisi dans le groupe constitué par les blocs de polyaminoacides, les blocs d'esters alkanolamines de l'acide polyméthacrylique, les blocs de polyamines, les blocs de polyvinylpyridine.

15. Composition de coloration selon l'une quelconque des revendications 1 à 14, dans laquelle le copolymère blocs est présent en une quantité allant de 0,001 à 20% en poids par rapport au poids total de la composition, de préférence, de 0,01 à 10% en poids.

16. Composition de coloration selon l'une quelconque des revendications 1 à 15, comprenant, en outre, au moins une base d'oxydation choisie parmi les paraphénylènediamines, les bis-phénylalkylènediamines, les para-aminophénols, les ortho-aminophénols et les bases hétérocycliques.

17. Composition de coloration selon l'une quelconque des revendications 1 à 16, comprenant, en outre, au moins un coupleur choisi parmi les métaphénylènediamines, les méta-aminophénols, les métadiphénols, les coupleurs naphtaléniques et les coupleurs hétérocycliques.

18. Composition de coloration selon l'une quelconque des revendications 1 à 17, présentant un pH allant de 4 à 11.

19. Composition de coloration selon l'une quelconque des revendications 1 à 18, comprenant, en outre, un agent oxydant.

20. Composition de coloration selon la revendication 19, dans laquelle l'agent oxydant est choisi parmi le peroxyde d'hydrogène, le peroxyde d'urée, les bromates de métaux alcalins, les persels tels que les perborates et persulfates, les peracides, les enzymes oxydases.

21. Composition de coloration selon l'une quelconque des revendications 1 à 20, comprenant, en outre, au moins un additif choisi parmi les tensioactifs anioniques, non-ioniques, amphotères, les polymères filmogènes anioniques, non ioniques différents des copolymères blocs tels que définis à la revendication 1, les polymères conditionneurs cationiques ou amphotères, les silicones linéaires, ramifiées ou cycliques, volatiles ou non, organomodifiées ou non, les épaississants polymères associatifs ou non, les épaississants non polymères, les agents nacrants, les opacifiants, les colorants ou les pigments, les parfums, les huiles minérales, végétales et/ou synthétiques, les cires, les vitamines, les filtres UV, les agents anti-radicalaires, les antipelliculaires, les conservateurs, les agents de stabilisation de pH, les solvants et leurs mélanges.

22. Procédé de coloration de fibres kératiniques comprenant l'application d'au moins une composition de teinture telle que définie selon l'une quelconque des revendications 1 à 21 sur des fibres sèches ou humides puis l'observation d'un temps de pause suivi éventuellement du rinçage des fibres.

23. Utilisation d'un copolymère blocs tel que défini selon l'une quelconque des revendications 1 à 21 pour améliorer la solubilité en milieu aqueux d'un colorant tel que défini selon l'une quelconque des revendications 1 à 21.

24. Utilisation d'un copolymère blocs tel que défini selon l'une quelconque des revendications 1 à 21 pour améliorer la stabilité en milieu oxydant et réducteur d'un colorant tel que défini selon l'une quelconque des revendications 1 à 21.

25. Utilisation d'une composition telle que définie selon l'une quelconque des revendications 1 à 21, pour améliorer l'unisson de la coloration sur des fibres kératiniques.
